# EUROPEAN PATENT APPLICATION

(11) **EP 4 317 176 A1**
(43) Date of publication of application: **07.02.2024**
(21) Application number: 22776058.4
(22) Date of filing: 22.03.2022
(51) Int. Cl.: C07K 14/005, C12N 15/86, A61K 39/00, A61P 31/14, A61P 35/00

(54) **ATTENUATED REOVIRUS-BASED VACCINE COMPOSITION AND USE THEREOF**

(30) Priority: 23.03.2021 KR 20210037160
(71) Applicant: Virocure, Inc., Seoul 08381 (KR)
(72) Inventor: YOO, Haeng Jun, Seoul 05698 (KR); ALAIN, Tommy Michel, Ottawa, Ontario K1W 1J2 (CA); HAN, Sang Kyoung, Seoul 06703 (KR); XIANG, Xiao, Ottawa, Ontario K1J 8P1 (CA); KIM, So Young, Seoul 08816 (KR); LEE, Yeon Sook, Gunpo-si Gyeonggi-do 15821 (KR); SONG, Ki Hoon, Paju-si Gyeonggi-do 10879 (KR)
(74) Representative: Dehns
(86) International application number: PCT/KR2022/003992
(87) International publication number: WO 2022/203358

(57) **Abstract**

The present invention relates to an attenuated reovirus-based vaccine composition and a use thereof, the attenuated reovirus, according to the present invention, having the 251st to 455th amino acids of a sigma-1 protein of a capsid truncated such that when an epitope of an antigenic protein inducing cancer or infectious disease is introduced to the truncated site of the sigma-1 protein, the epitope of the antigenic protein is stably expressed in a cell, and thus the effect is gained of exhibiting an immune response such as producing a neutralizing antibody or inducing cell-mediated immunity. As such, the present invention is expected to be usefully employable as a vaccine composition for cancer or infectious disease by introducing the epitope of the antigenic protein to the truncated site of the sigma-1 protein of the attenuated reovirus according to the present invention.

## Description

### [Technical Field]

The present invention relates to an attenuated reovirus-based vaccine composition and use thereof.

This application claims priority based on Korean Patent Application No. 10-2021-0037160 filed on March 23, 2021, and Korean Patent Application No. 10-2022-0035224 filed on March 22, 2022. All contents disclosed in the specifications and drawings of these applications are incorporated herein by reference.

### [Background Art]

A virus is a small non-cellular organism composed of genetic material and protein, and there exist different types of viruses. For example, a virus can be a DNA virus that replicates within the nucleus of a host or an RNA virus that replicates within the cytoplasm of a cell. A virus can be double-stranded or single-stranded. Moreover, a single-stranded RNA virus can be positive (+, sense) stranded or negative (-) stranded. These different types of viruses cause various viral infections.

Viral infections occur when a pathogenic virus enters an organism. Once the virus enters an organism, the virus attaches itself to a cell, reprograms the cell to replicate new viruses until the cell bursts or dies, allowing the virus to cause various infectious diseases in humans and animals and spread rapidly. Infectious diseases caused by viruses typically include colds, flu, and warts. However, viruses can also cause severe diseases such as acquired immunodeficiency syndrome (AIDS), coronavirus disease-19 (COVID-19), hepatitis C, tuberculosis, severe acute respiratory syndrome (SARS), Middle East respiratory syndrome (MERS), smallpox, herpes, hemorrhagic fever, polio, measles, mumps, rubella, infantile infantile caused by rotavirus, and non-bacterial acute gastroenteritis caused by norovirus.

Today, the most important protective measure against viral infections and limiting their spread is vaccination. Modern vaccines principally induce the formation of antibodies against surface virus antigens. The efficacy of a vaccine directly depends on the match between the antigenic structure of the virus contained in the vaccine and the strains circulating in the population. The surface proteins of most viruses undergo consistent antigenic variation (antigenic drift), necessitating continuous updating of vaccine strains. Developing a safe vaccine with high immunogenicity that induces a broad immune response is one of the main challenges faced in effective prevention of cancer or an infectious disease. There is a real need for vaccine development to prevent the spread and ongoing infections of global pandemic diseases like COVID-19 caused by SARS-CoV-2. Therefore, many virus-based vaccines are being developed globally using platforms such as vesicular stomatitis virus (VSV), measles virus (MeV), adenovirus (Ad), baculovirus, and the like.

The Reovirus (Respiratory Enteric Orphan Virus) is a non-enveloped virus comprising an entire genome of 10 double-stranded RNA fragments, ubiquitous in common environments, and is an asymptomatic virus that does not show symptoms in hosts with normal immune functions. Infections in humans are known to be mild and are believed to be confined to the upper respiratory and gastrointestinal tracts.

Meanwhile, reverse genetics technology involves verifying the entire genomic sequence of a virus to produce a recombinant artificial virus. The greatest advantage is that the virus can be recombinantly expressed, manipulated, and synthesized artificially, allowing various manipulations to be applied to the virus genes as intended. New viruses modified by loading various mechanisms, such as chimeric virus, codon pair deoptimization, and mutagenesis, onto the genes of the virus can be produced. The classical genetics approach mainly involves randomly inducing mutations in the genome of a given organism and then discovering genes from the mutations that exhibit the trait of interest, which is time-consuming and laborious and has the disadvantage that it is not easy to isolate mutations in genes that do not have strong trait modifying effects. With the recent development of functional genomics, genome-level mutations have become available in several model organisms, including yeast. In contrast, reverse genetics technology can increase the identification capacity of genetic exploration by directly introducing and exploring potential trait variations that each gene's mutation can cause. The reverse genetics technology has the advantage of being able to manipulate the virus artificially as desired and maximize its characteristics, making it a very useful technology in vaccine development.

In this regard, the inventors of the present invention aimed to develop a vaccine platform based on reoviruses by using reverse genetics technology to insert an epitope sequence into the attenuated reovirus sigma-1 protein by a "quick exchange" method in which a specific amino acid sequence position is truncated to fuse and express an epitope for cancer or an infectious disease into the attenuated reovirus sigma-1 protein.

### [Disclosure]

### [Technical Problem]

The inventors of the present invention have found that in the attenuated reovirus sigma-1 protein where amino acids 251 to 455 are truncated, by introducing the amino acid sequence of various epitopes for cancer or an infectious disease at the site of the truncated amino acids via substitution or insertion, the introduced epitope is stably expressed in the cell. This expression results in generating neutralizing antibodies or inducing cell-mediated immunity, thus manifesting an immune response. Based on this, the present invention was completed.

Accordingly, the present invention is directed to providing a polypeptide comprising the amino acid sequence of the attenuated reovirus sigma-1 protein; and the amino acid sequence of an epitope that induces cancer or an infectious disease, and a polynucleotide encoding the same.

The present invention is also directed to providing a virus vector based on the attenuated reovirus, comprising the polynucleotide encoding the polypeptide.

The present invention is also directed to providing a vaccine composition based on the attenuated reovirus, comprising the polypeptide, the polynucleotide encoding the same, or the virus vector as an active ingredient, and a method of preparing the same.

However, technical problems to be solved in the present invention are not limited to the above-described problems, and other problems which are not described herein will be fully understood by those of ordinary skill in the art from the following descriptions.

### [Technical Solution]

To achieve the purposes of the present invention, the present invention provides a polypeptide comprising an amino acid sequence of an attenuated reovirus sigma-1 protein; and an amino acid sequence of an epitope of an antigen causing cancer or an infectious disease,
wherein the polypeptide comprises the 1 to 250th amino acid sequence of the attenuated reovirus sigma-1 protein truncated at the 251st amino acid position from the N-terminus; and
an amino acid sequence of an epitope of an antigen causing cancer or an infectious disease inserted at the amino acid position including and subsequent to the 251st from the N-terminus of the amino acid sequence of the attenuated reovirus sigma-1 protein.

Moreover, the present invention provides a virus vector based on the attenuated reovirus comprising the polynucleotide encoding the polypeptide.

Additionally, the present invention provides a vaccine composition based on the attenuated reovirus, comprising the polypeptide, the polynucleotide encoding the same, or the virus vector as an active ingredient.

Furthermore, the present invention provides a method of preparing a vaccine composition based on the attenuated reovirus, comprising the following steps:
a) preparing a reverse genetics virus vector comprising a base sequence encoding an attenuated reovirus sigma-1 protein represented by SEQ ID NO: 1; and
b) introducing a polynucleotide into the vector, wherein the polynucleotide encodes an attenuated reovirus sigma-1 protein and an epitope of an antigen causing cancer or an infectious disease, which is expressed as a fusion, by substituting or inserting a base at positions 763 to 1416 of the base sequence encoding the sigma-1 protein, so as to comprise a base sequence encoding the epitope of the antigen causing cancer or an infectious disease.

In one embodiment of the present invention, the attenuated reovirus sigma-1 protein may be represented by the amino acid sequence of SEQ ID NO: 1, but is not limited thereto.

In yet another embodiment of the present invention, the attenuated reovirus sigma-1 protein may be encoded by the base sequence represented by SEQ ID NO: 2, but is not limited thereto.

In yet another embodiment of the present invention, the epitope may form or constitute a fusion protein at the carboxy-terminus of the attenuated reovirus sigma-1 protein, but is not limited thereto.

In yet another embodiment of the present invention, one or more amino acid sequence selected from the group consisting of a linker (SEQ ID NO: 11), Myc protein (SEQ ID NO: 12), FLAG protein (SEQ ID NO: 13), and 2A peptide may further be comprised, both before and after the amino acid sequence of the epitope, but is not limited thereto.

In yet another embodiment of the present invention, the cancer may be one or more selected from the group consisting of liver cancer, glioma, sarcoma, colon cancer, breast cancer, prostate cancer, melanoma, lung cancer, head and neck cancer, ovarian cancer, bladder cancer, stomach cancer, esophageal cancer, bile duct cancer, pancreatic cancer, cervical cancer, skin cancer, lymphoma, thyroid cancer, bone marrow cancer, endometrial cancer, kidney cancer, rectal cancer, and brain tumor, but is not limited thereto.

In yet another embodiment of the present invention, the infectious disease may be one or more selected from the group consisting of coronavirus disease-19 (COVID-19), hepatitis C, influenza, human immunodeficiency virus (HIV)-induced acquired immune deficiency syndrome (AIDS), tuberculosis, severe acute respiratory syndrome (SARS), Middle East respiratory syndrome (MERS), infantile enteritis caused by rotavirus, and non-bacterial acute gastroenteritis caused by norovirus, but is not limited thereto.

In yet another embodiment of the present invention, the amino acid sequence of the epitope may be one or more selected from the group consisting of SEQ ID NO: 3 to 10, but is not limited thereto.

In yet another embodiment of the present invention, the base sequence encoding the epitope may be substituted or inserted at positions 763 to 1416 from the 5' end of the base sequence encoding the attenuated reovirus sigma-1 protein, but is not limited thereto.

In yet another embodiment of the present invention, the epitope may induce an immune response by producing neutralizing antibodies or inducing cell-mediated immunity in a host, but is not limited thereto.

In yet another embodiment of the present invention, the epitope may be one or more selected from the group consisting of CD4+ T cell, CD8+ T cell, and B-cell epitopes, but is not limited thereto.

In yet another embodiment of the present invention, the vaccine composition may be for preventing or treating cancer or an infectious disease, but is not limited thereto.

In yet another embodiment of the present invention, in the method of preparing the vaccine composition, it may comprise the step of c) producing and proliferating the vector based on the attenuated reovirus comprising the polynucleotide from the step b) in one or more cell selected from the group consisting of BHK21, L929, HEK293, CHO, PER.C6, HeLa, and Vero cells, but is not limited thereto.

In addition, the present invention provides a method for preventing or treating cancer or an infectious diseases comprising administering the vaccine composition to a subject in need thereof.

Moreover, the present invention provides a use of the vaccine composition for preventing or treating cancer or an infectious disease.

Furthermore, the present invention provides a use of the polypeptide according to the present invention, a polynucleotide encoding the polypeptide, or the virus vector for preparing a vaccine for preventing or treating cancer or an infectious disease.

### [Advantageous Effects]

The attenuated reovirus according to the present invention is one in which 251 to 455th amino acids of the sigma-1 protein of the capsid are truncated, such that when an epitope of an antigenic protein causing cancer or an infectious disease is introduced into the truncated site of the sigma-1 protein, the epitope of the antigenic protein is stably expressed in the cell and has the ability to produce an immune response, such as producing neutralizing antibodies or inducing cell-mediated immunity. Accordingly, by introducing the epitope of the antigenic protein into the truncated site of the attenuated reovirus sigma-1 protein according to the present invention, it is anticipated to be beneficially used as a vaccine composition for cancer or an infectious disease.

### [Brief Description of the Drawings]

FIG. 1A represents a comparison of the structures of the wild-type reovirus and the attenuated reovirus RP116 according to an embodiment of the present invention.
FIG. 1B represents a drawing showing that epitopes of various pathogen-derived antigens can be added to the truncated site of the sigma-1 protein of the attenuated reovirus RP116 according to an embodiment of the present invention to create a reovirus vaccine platform.
FIG. 1C represents a drawing depicting the route of administration and action of a vaccine prepared using the attenuated reovirus according to an embodiment of the present invention.
FIG. 2 represents a drawing illustrating the quick exchange reovirus reverse genetics system and the reovirus replication process according to an embodiment of the present invention.
FIG. 3A represents a drawing verifying the protein detection results after infecting L929 cells with wild-type reovirus (WT ReoV), attenuated reovirus RP116 with truncated σ1 (ReoV+Q251*), and reovirus introduced with RBD (ReoV+RBD) based on anti-reovirus antibodies according to an embodiment of the present invention.
FIG. 3B represents a drawing verifying the detection of the RBD gene from the genetic material extracted from L929 cells infected with ReoV+RBD according to an embodiment of the present invention.
FIG. 3C represents a drawing verifying the expression of the reovirus protein and RBD based on the SARS-CoV-2 neutralizing antibody (NeuAb) (top) and anti-reovirus antibody (bottom) in the cell lysate obtained from L929 cells infected with ReoV+RBD according to an embodiment of the present invention.
FIG. 4A represents a drawing depicting a construct design of the recombinant σ1 protein of the attenuated reovirus according to an embodiment of the present invention.
FIG. 4B represents a drawing verifying the expression of various CD4+ and CD8+ T cell epitopes fused with σ1 protein in the attenuated reovirus according to an embodiment of the present invention through Western blot analysis.
FIG. 4C represents a drawing verifying the expression of introduced antigens in the recombinant reovirus used in FIG. 4B through RT-qPCR analysis.
FIG. 4D represents a drawing verifying the expression of various B-cell epitopes fused with σ1 protein in the attenuated reovirus according to an embodiment of the present invention through Western blot analysis.
FIG. 4E represents a drawing verifying the expression of introduced antigens in the recombinant reovirus used in FIG. 4D through RT-qPCR analysis.
FIG. 5 represents a drawing verifying the epitope expression in ReoV+OVA²⁵⁷⁻²⁶⁴ according to an embodiment of the present invention through immunocytochemistry analysis.
FIG. 6 represents a drawing verifying the expression of the linear B-cell epitope of SARS-CoV-2 in ReoV-S21P2(2) according to an embodiment of the present invention.
FIG. 7A represents a drawing depicting a construct design of the recombinant σ1 protein of the recombinant reoviruses ReoV+S21P2(1) and ReoV+OVA²⁵⁷⁻²⁶⁴ according to an embodiment of the present invention.
FIG. 7B represents a drawing verifying the stability of the introduced epitope by detecting antigens with reovirus-specific antibodies (top) and specific antibodies against the FLAG tag (bottom) in the recombinant reovirus according to an embodiment of the present invention.

### [Best Modes of the Invention]

In one embodiment of the present invention, an attenuated reovirus RP116 with a unique STOP mutation from nucleotide ⁷⁶³CAA (amino acid ²⁵¹Q-Glutamine) to ⁷⁶³TAA (STOP codon) is used to add an epitope amino acid sequence to the truncated sigma-1 protein of RP116 and establish a vaccine platform based on the attenuated reovirus through a quick exchange reovirus reverse genetics system (refer to Example 1 and 2).

In another embodiment of the present invention, as a proof of concept for the reovirus vaccine platform, ReoV-RBD expressing a codon-optimized sequence of the receptor binding domain (RBD) of SARS-CoV-2 (Severe Acute Respiratory Syndrome Coronavirus 2) in fusion with the sigma-1 protein of the reovirus was prepared, and it could be detected by SARS-CoV-2 neutralizing antibodies. So, it indicates that ReoV expressing the RBD generates ideal neutralizing antibodies as a vaccine candidate (refer to Example 3).

In yet another embodiment of the present invention, the quick exchange technique was used to be manipulated to express various CD4+ and CD8+ T cell epitopes in fusion with the attenuated reovirus sigma-1 protein. It was confirmed through experiments that the epitopes are indeed expressed (refer to Example 4 to 6), and that the introduced epitopes can be stably expressed without mutations (refer to Example 7).

Thus, the present invention provides a polypeptide comprising an amino acid sequence of an attenuated reovirus sigma-1 protein; and an amino acid sequence of an epitope of an antigen causing cancer or an infectious disease,
wherein the polypeptide comprises the 1 to 250th amino acid sequence of the attenuated reovirus sigma-1 protein truncated at the 251st amino acid position from the N-terminus; and
an amino acid sequence of an epitope of an antigen causing cancer or an infectious disease inserted at the amino acid position including and subsequent to the 251st from the N-terminus of the amino acid sequence of the attenuated reovirus sigma-1 protein.

As used herein, the term "reovirus (respiratory enteric orphan virus, REO virus)" refers to a virus with 20 faces, non-enveloped, possessing double-stranded RNA fragments as its genome. The reovirus is commonly isolated from the digestive and respiratory systems of healthy humans and is considered a non-pathogenic virome. The reovirus is known as an oncolytic virus capable of infecting and destroying various transformed cells.

As used herein, the term "attenuated reovirus" may be the reovirus RP1 16 with a mutation from ⁷⁶³CAA, which encodes the 251st amino acid, Glutamine (Q), in the attachment sigma-1 protein exposed on the wild-type reovirus capsid, to ⁷⁶³TAA (STOP codon), resulting in a truncation after the 251st amino acid of the sigma-1 protein, thereby leading to the truncation of the spherical head shape.

In the present invention, the attenuated reovirus sigma-1 protein may be represented by the amino acid sequence of SEQ ID NO: 1, but is not limited thereto.

In the present invention, the attenuated reovirus sigma-1 protein may be encoded by the base sequence represented by SEQ ID NO: 2, but is not limited thereto.

As used herein, the term "cancer" refers to a disease caused by cells that ignore normal growth boundaries and exhibit aggressive characteristics of division and growth, invasive characteristics of penetrating surrounding tissues, and metastatic characteristics of spreading to other parts of the body.

In the present invention, the cancer is not particularly limited if it is known as a malignant tumor in the art, and may be one or more selected from the group consisting of liver cancer, glioma, sarcoma, colon cancer, breast cancer, prostate cancer, melanoma, lung cancer, head and neck cancer, ovarian cancer, bladder cancer, stomach cancer, esophageal cancer, bile duct cancer, pancreatic cancer, cervical cancer, skin cancer, lymphoma, thyroid cancer, bone marrow cancer, endometrial cancer, kidney cancer, rectal cancer, and brain tumor.

As used herein, the term "infection" means the invasion, development, and proliferation of pathogenic microorganisms like viruses in the body of a host organism, and refers to their settlement and proliferation on the tissues, body fluids, and surfaces of humans, animals, or plants. As a result, the host may undergo pathological changes and may develop a disease.

As used herein, the term "infectious disease" refers to a disease caused by pathogenic microorganisms settling and proliferating on the tissues, body fluids, and surfaces of humans, animals, or plants and may be classified into several types depending on the route of infection and contagiousness. The infections comprise viral infections, fungal infections, bacterial infections, protozoan infections, and parasitic infections. In the present invention, the infectious disease may be at least one selected from the group consisting of hepatitis C, influenza, human immunodeficiency virus (HIV)-induced acquired immune deficiency syndrome (AIDS), tuberculosis, coronavirus disease-19 (COVID-19), severe acute respiratory syndrome coronavirus 2 (SARS-CoV-2) infection, infantile enteritis caused by rotavirus, and non-bacterial acute gastroenteritis caused by norovirus, and in an embodiment of the present invention, it may be COVID-19, but is not limited thereto.

In the present invention, "coronavirus disease-19 (COVID-19)" is a severe respiratory syndrome caused by SARS-CoV-2. The first case was reported in China in December 2019, and it spread globally becoming a pandemic. The symptoms of COVID-19 vary but include fever, cough, headache, fatigue, difficulty breathing, and loss of smell and taste. Symptoms appear within 1-14 days of being infected. Notably, one-third of infected individuals are asymptomatic and show no noticeable symptoms. Eighty-one percent of people who are noticeable enough to be classified as patients develop mild to moderate symptoms, 14% develop symptoms such as shortness of breath and hypoxia, and 5% develop severe symptoms such as respiratory failure and shock. Older adults are more likely to develop severe symptoms, and organ damage has been observed in some people due to prolonged exposure to COVID-19 after recovery.

SARS-CoV-2 comprises 30 kb of nucleotide and have four important structural proteins; Nucleocapsid (N), Spike (S), Membrane (M), and Envelope (E) proteins. Among them, the S protein represents a critical site for binding with the host cell's receptor and delivers the viral nucleocapsid into the cell where replication takes place.

The most straightforward and direct method for combating SARS-CoV-2 is to neutralize the virus entering human cells, thereby blocking the mechanism where SARS-CoV-2 infiltrates the cell, replicates, and new virions are secreted to infect other cells.

SARS-CoV-2 is known to be able to replicate by binding to the angiotensin converting enzyme 2 (ACE2) receptor on human cells. The receptor-binding domain (RBD) of the S protein of coronavirus is a key region that binds to the ACE2 receptor on host cells. It contains multiple conformational-dependent epitopes that induce potent neutralizing antibodies against SARS-CoV-2 infection. Thus, it can be a crucial target for the treatment and vaccine development against COVID-19 (J. Immunol 2005;174:4908-4915).

Furthermore, the present invention provides a virus vector based on attenuated reovirus, comprising a polynucleotide encoding the polypeptide.

Additionally, the present invention provides a vaccine composition based on the attenuated reovirus, comprising the polypeptide, the polynucleotide encoding the same, or the virus vector as an active ingredient.

In the present invention, the vaccine composition may be for preventing or treating cancer or an infectious disease, but is not limited thereto.

In the present invention, the term "vaccine" refers to a composition comprising at least one immunologically active ingredient that induces an immunological response in animals. The immunologically active ingredient of the vaccine may comprise appropriate elements of a living or dead virus (subunit vaccine), and these elements may be obtained by destroying the entire virus or its growth culture, followed by a purification process, or, but not limited to, a synthetic process induced by appropriate manipulation of suitable systems such as bacteria, insects, mammals, or other species, followed by a separation and purification process, or by directly incorporating genetic material into animals that require the vaccine by using a suitable pharmaceutical composition (polynucleotide vaccination).

The vaccine may comprise one or more of the elements described above and may be prepared by methods known in the art. In the present invention, the vaccine may be manufactured based on an attenuated reovirus with a truncated sigma-1 protein and may be designed to express various epitopes that cause cancer or an infectious disease in a fused form at its truncated site. For example, it may be manufactured in the form of a polypeptide comprising the amino acid sequence of the truncated sigma-1 protein of the attenuated reovirus and the epitope, a polynucleotide encoding the same, and a virus vector comprising the polynucleotide, but is not limited thereto. The vaccine of the present invention may be in any form known in the art, for example, in the form of a liquid or injection, or in a solid form suitable for a suspension, but is not limited thereto. Such formulations can also be emulsified or encapsulated within liposomes or soluble glasses, or may be manufactured in the form of aerosols or sprays. They may also be included in transdermal patches.

According to the present invention, the vaccine may, if necessary, comprise a pharmaceutically acceptable vaccine preservative, an adjuvant, a diluent, an absorption enhancer, and the like. The vaccine preservative may comprise, for example, a lactose phosphate glutamate gelatin mixture, but is not limited thereto. In the case where the vaccine is a liquid or an injectable, it can comprise, if necessary, propylene glycol and a sufficient amount (e.g., about 1%) of sodium chloride to prevent hemolysis.

The substance used as the adjuvant is not particularly limited and may, for example, be Alum, monophosphoryl lipid A (MPL), aluminum hydroxide, mineral oil or other oil or an additive to the vaccine, or may be an auxiliary molecule produced by the body after being induced by such additional ingredients, but is not limited thereto.

In this context, "pharmaceutically acceptable" means it is physiologically acceptable and, when administered to a subject, does not inhibit the action of an active ingredient and typically does not cause allergic reactions such as gastrointestinal disorders or dizziness, implying non-toxicity.

The vaccine may be administered through routes of administration such as oral, transdermal, intramuscular, intraperitoneal, intravenous, subcutaneous, and the like, but is not limited thereto, and for example, may be administered orally or intramuscularly.

As used herein, the term "epitope" refers to an antigenic determinant that allows the immune system, such as antibodies, B cells, and T cells, to recognize an antigen, specifying a particular portion of the antigen. To induce a B cell (i.e., antibody) response, the antigen must comprise a "B cell epitope", and to induce a T cell response, the antigen must comprise a "T cell epitope". As commonly understood in the art, a B cell epitope is a part of the antigen recognized and bound by a B cell receptor. Lipids, polysaccharides, and protein/peptides can comprise B cell epitopes, and when introduced into a selected organism, they prompt the B cell to produce antibodies that specifically bind to the introduced epitope.

In the present invention, the epitope may be one or more selected from the group consisting of CD4+ T cells, CD8+ T cells, and B-cell epitopes, and according to an embodiment of the present invention, may comprise one or more amino acid sequence selected from the group consisting of RBD (SEQ ID NO: 3), OVA²⁵⁷⁻²⁶⁴ (SEQ ID NO: 4), OVA³²³⁻³³⁹ (SEQ ID NO: 5), Adpgk (SEQ ID NO: 6), Rpl18 (SEQ ID NO: 7), P15E (SEQ ID NO: 8), S21P2(1) (SEQ ID NO: 9), and S21P2(2) (SEQ ID NO: 10), but is not limited thereto.

In the present invention, the epitope may form or constitute a fusion protein at the carboxy-terminus of the attenuated reovirus sigma-1 protein, but is not limited thereto.

In the present invention, the base sequence encoding the epitope may be substituted or inserted at positions 763 to 1416 from the 5' end of the base sequence encoding the attenuated reovirus sigma-1 protein, but is not limited thereto. For example, the base sequence encoding the epitope may substitute for a specific base sequence between position 763 to 1416 from the 5' end of the base sequence of SEQ ID NO: 2 encoding the attenuated reovirus sigma-1 protein, and may also be inserted between specific nucleotide sequences. Accordingly, in the attenuated reovirus sigma-1 protein after the 251st amino acid sequence from the N-terminus of the wild-type reovirus sigma-1 protein is truncated, the epitope that is inserted and fused into the amino acid position including and after the 251st position in the attenuated reovirus sigma-1 protein may comprise an amino acid sequence of about 5 to 400, but is not limited thereto.

In the present invention, the attenuated reovirus sigma-1 protein may further comprise at least one amino acid sequence selected from the group consisting of a linker (SEQ ID NO: 11), Myc protein (SEQ ID NO: 12), FLAG protein (SEQ ID NO: 13), and a 2A peptide, both before and after the amino acid sequence of the epitope included at the amino acid position including and subsequent to the 251st, but not limited thereto, and there is no limitation to the order of these. The 2A peptide may be selected from 2A peptides comprising, for example, P2A, T2A, E2A, or F2A, and there are no limitations to its types.

In the present invention, one or more epitope selected from the group consisting of CD4+ T cell, CD8+ T cell, and B-cell epitopes may cause an immune response by allowing T cells or B cells to identify the antigen, leading to the generation of neutralizing antibodies in the host or inducing cell-mediated immunity, but is not limited thereto.

As used herein, the term "antibody" refers to a polypeptide that comprises a framework region derived from immunoglobulin genes or their fragments and specifically recognizes and binds to an antigen. The recognized immunoglobulin genes include kappa, lambda, alpha, gamma, delta, epsilon, and mu constant region genes, as well as numerous immunoglobulin variable region genes. Light chains are classified as kappa or lambda. Heavy chains are classified as gamma, mu, alpha, delta, or epsilon, thus defining immunoglobulin classes IgG, IgM, IgA, IgD, and IgE, respectively. Typically, the antigen-binding region of an antibody becomes crucial in binding specificity and affinity. Antibodies are the main actors of humoral immunity and, together with T cells, play a vital role in the body's defense mechanisms and are prepared by administering antigenic proteins as immunogens to animals to induce a humoral immune response. The antibody or fragments of the antibody may be derived from various subjects, including humans, mice, rats, hamsters, camels, rabbits, etc., but is not limited thereto.

As used herein, the term "neutralizing antibody" refers to any antibody or its antigen-binding fragment that binds to a pathogen and interferes with the pathogen's ability to infect cells or cause disease.

As used herein, the term "cell-mediated immunity" refers to an immune activity in which a cell of lymphatic system directly induces an immune response to a particular antigen. This immune activity refers to the phagocytic action where a white blood cell acts upon an antigen to ingest the cell, or induces a cytotoxic cell reaction to eliminate the antigen. This is a component of the immune system, in conjunction with humoral immunity performed by serum antibodies, and notably includes the action of T cells that possess cytotoxicity. The T cells bind with B cells to form antibodies and then directly come into contact with and destroy the antigen.

As used herein, the term "antigen" represents a protein capable of inducing an immune response in a host. The antigen can be recognized and bound by an antibody. An antigen can originate either within the body or from an external environment and includes recombinant forms of proteins. In the present invention, the vaccine composition is capable of inducing the production of antibodies against the epitope of the antigen causing cancer or an infectious disease, wherein the epitope acts as an antigen. The production of antibodies may be more increased when the vaccine composition is administered twice or three times compared to a single administration, but is not limited thereto.

Moreover, the present invention provides a method of preparing a vaccine composition based on the attenuated reovirus, comprising the following steps:
a) preparing a reverse genetics virus vector comprising a base sequence encoding an attenuated reovirus sigma-1 protein represented by SEQ ID NO: 1; and
b) introducing a polynucleotide into the vector, wherein the polynucleotide encodes an attenuated reovirus sigma-1 protein and an epitope of an antigen causing cancer or an infectious disease, which is expressed as a fusion, by substituting or inserting a base at positions 763 to 1416 of the base sequence encoding the sigma-1 protein, so as to comprise a base sequence encoding the epitope of the antigen causing cancer or an infectious disease.

In the present invention, the reverse genetics virus vector may refer to a virus vector prepared to produce the S1 segment RNA encoding the attenuated reovirus sigma-1 protein in host cells and can be prepared by methods known in the art. The vector is structured to allow the virus RNA segment to be transcribed from the T7 RNA polymerase promoter, and the 3' end naturally forms by a ribozyme present in the vector. In cells expressing the T7 polymerase where the vector is introduced, the virus RNA is produced, and using this, the virus protein is synthesized, thus termed the reverse genetics system. This is a useful method for designing and producing mutants of RNA viruses.

In the method for preparing the vaccine composition of the present invention, the step c) comprises producing and proliferating the vector based on the attenuated reovirus comprising the polynucleotide from the step b) in one or more cell selected from the group consisting of BHK21, L929, HEK293, CHO, PER.C6, HeLa, and Vero cells, but is not limited thereto.

In addition, the present invention provides a method for preventing or treating cancer or an infectious diseases comprising administering the vaccine composition to a subject in need thereof.

Moreover, the present invention provides a use of the vaccine composition for preventing or treating cancer or an infectious disease.

Furthermore, the present invention provides a use of the polypeptide according to the present invention, a polynucleotide encoding the polypeptide, or the virus vector for preparing a vaccine for preventing or treating cancer or an infectious disease.

The term "prevention" as used herein means all actions that inhibit or delay the onset of a target disease. The term "treatment" as used herein means all actions that alleviate or beneficially change a target disease and abnormal metabolic symptoms caused thereby via administration of the pharmaceutical composition according to the present invention.

As used herein, the term "administration" refers to providing a subject with a predetermined composition of the present invention by using an arbitrary appropriate method.

As used herein, the term "subject" refers to a subject in need of prevention of cancer or an infectious disease, and more specifically, refers to a mammal such as a human or a non-human primate, a mouse, a dog, a cat, a horse, and a cow.

In the present invention, when the term "comprising" is used, unless specifically contradicted, it does not mean excluding other components but may include other components. The degree term used throughout the present invention "step of ~" or "step of the" does not mean "step for ~".

Hereinafter, preferable examples are presented to aid in the understanding of the present invention. However, these examples are provided merely for easier understanding of the present invention, and the content of the present invention is not limited by these examples.

### [Examples]

### Example 1. Construction of Attenuated Reovirus-based Vaccine Platform

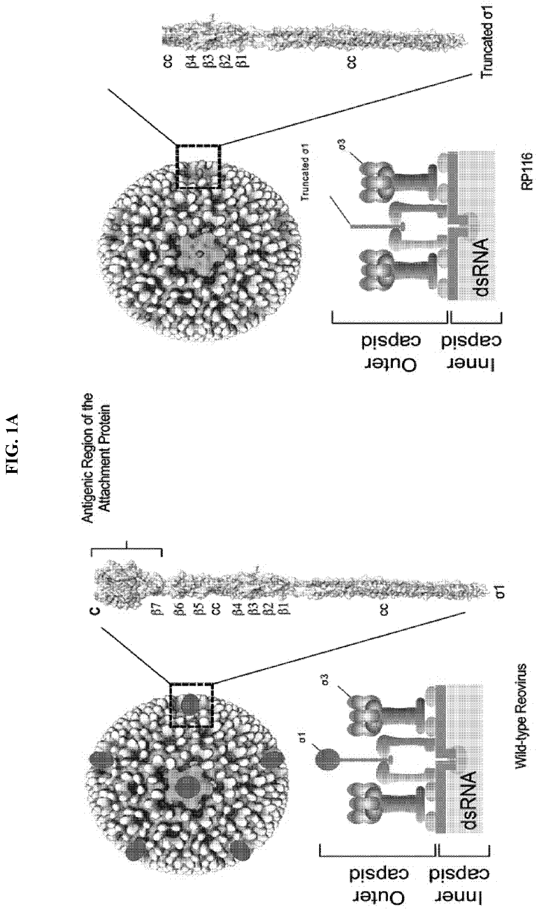

Reovirus is a double-stranded RNA non-enveloped virus. REO is an abbreviation for Respiratory Enteric Orphan, which was isolated for human respiratory and intestinal systems but is not associated with any known human diseases. Most of the neutralizing antibodies and immune responses caused by wild-type reovirus infection are directed against the sigma-1(σ1) protein of the virus, more specifically, against the spherical head of the protein that protrudes from the capsid. Therefore, the region of sigma-1 in the reovirus represents a key part enhancing its antigenicity. As shown in FIG. 1A, the wild-type (WT) reovirus represents the attachment protein σ1 on the external capsid that recognizes and binds to the Junctional Adhesion Molecule-A (JAM-A) (left drawing of FIG. 1A).

The amino acid sequence and base sequence of the wild-type reovirus σ1 protein are shown in Table 1.

**[Table 1]**

| | Sequence | SEQ NO: |
|---|---|---|
| RP116 sigma-1 protein | MDPRLREEVVRLIIALTSDNGVSLSKGLESRVSALEK TSQIHSDTILRITQGLDDANKRIIALEQSRDDLVASVS DAQLAISRLESSIGALQTVVNGLDSSVTQLGARVGQL ETGLAELRVDHDNLVARVDTAERNIGSLTTELSTLTLR VTSIQADFESRISTLERTAVTSAGAPLSIRNNRMTMGL NDGLXLSGNNLAIRLPGNTGLNIQNGGLQFRFNTDQ FQIVNNNLTLKTTVFDSINSRXGAXE | 1 |
| RP116 sigma-1 gene | TGAACTCCGACATTTTTATTGTAGATGATTACATACA TATATGTCTTCCAGCTTTTGACGGTTTCTCTATAGCT GACGGTGGAGATCTATCGTTGAACTTTGTTACCGG ATTGTTACCACCGTTACTTACAGGAGACACTGAGC CCGCTTTTCATAATGACGTGGTCACATATGGAGCAC AGACTGTAGCTATAGGGTTGTCGTCGGGTGGTGCG CCTCAGTATATGAGTAAGAATCTGTGGGTGGAGCA GTGGCAGGATGGAGTACTTCGGTTACGTGTTGAGG GGGGTGGCTCAATTACGCACTCAAACAGTAAGTGG CCTGCCATGACCGTTTCGTACCCGCGTAGTTTCACG TGAGGATCAGACCACCCCGCGGCACTGGGGCATTT CATC | 2 |

Attenuated reovirus RP116 is a reovirus with a unique STOP mutation from nucleotide ⁷⁶³CAA (amino acid ²⁵¹Q-Glutamine) to ⁷⁶³TAA (STOP codon), producing a "head-truncated" σ1 protein compared to WT reovirus σ1 (right drawing of FIG. 1A).

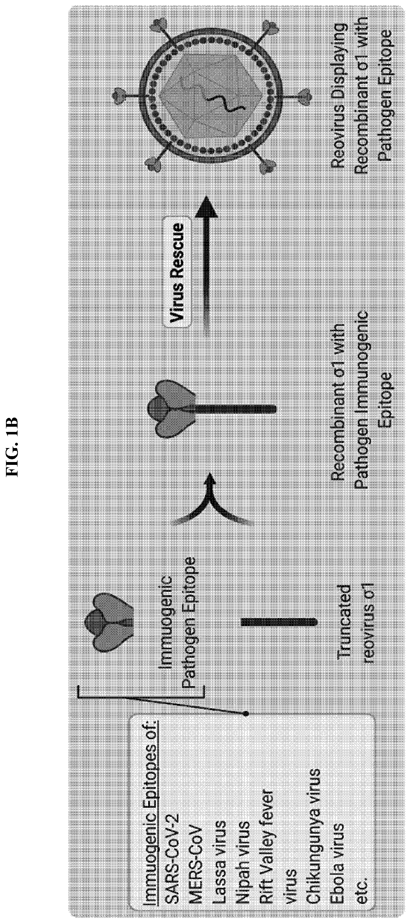

As shown in FIG. 1B, such σ1 protein of RP116 shows that the spherical head is not essential for reovirus replication, and fragments from various pathogens may be exchanged at this site. For the creation of an innovative and safe reovirus vaccine platform, approximately 5 to 400 amino acids of epitope fragments may be added to this site of RP116 σ1 protein.

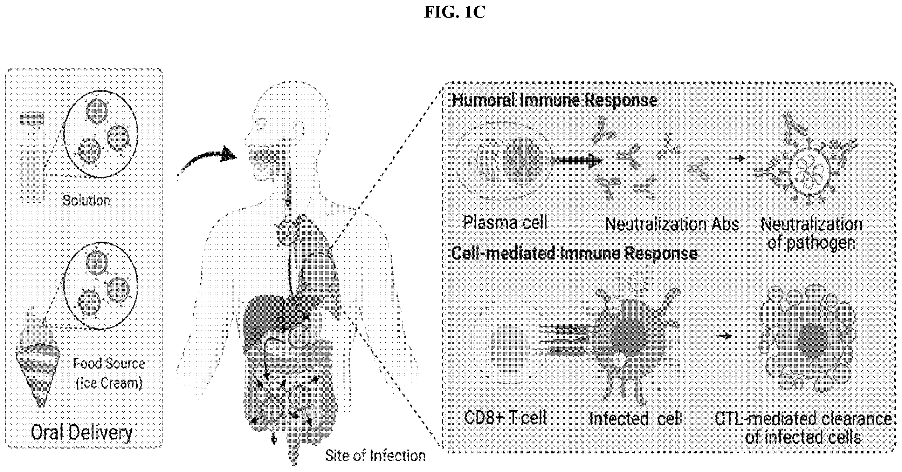

Also, as shown in FIG. 1C, non-enveloped reoviruses have resilience against harsh environmental conditions and can be administered with various solutions or food sources. Upon oral administration, the reovirus preferentially infects the M cells of the small intestine and induces an immune response against various pathogen epitopes represented on σ1.

### Example 2. Establishment of Quick Exchange Reovirus Reverse Genetics System

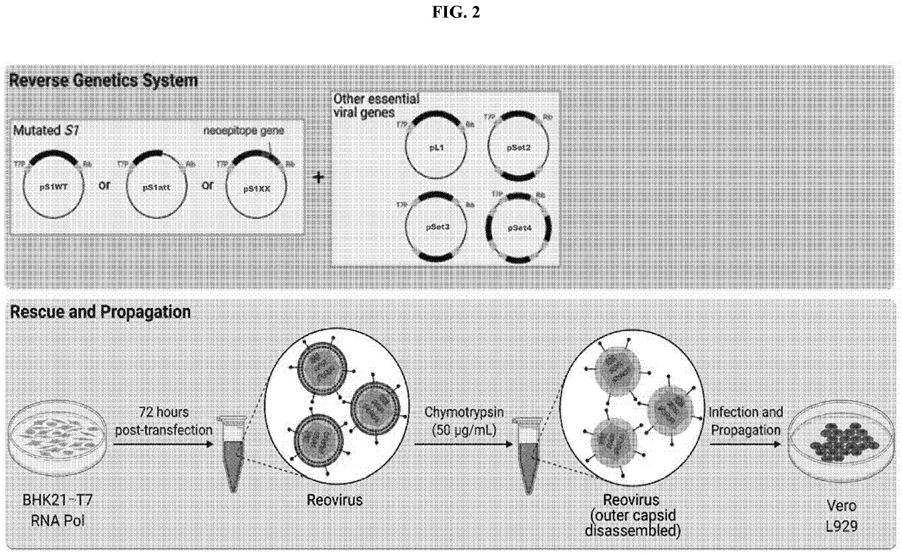

A reverse genetics system for the reovirus was transfected with 10 viral gene segments and a T7 expressing cell line to generate replicable reovirus particles (Kobayshi et al., 2007, Cell Host Microbe. 2007 Apr 19;1(2): 147-57.), then reported an improved version of the reverse genetics system in which only four plasmids containing 10 viral gene segments needed to be transfected into a T7 expressing cell line (Kobayashi et al., 2010, Virology. 2010 Mar 15; 398(2): 194-200.). In the present invention, a novel reverse genetics system was constructed using the attenuated reovirus RP116 gene, enabling a rapid "quick exchange" approach for vaccine design and testing. The vectors used to design the reovirus RP116-based vaccine by introducing the system in the present invention are as follows (refer to the top drawing of FIG. 2): pS1Att or pS1XX, pL1, pSet2, pSet3, pSet4.

When the vectors are introduced into BHK21 cells transformed to express the T7 RNA polymerase, the virus genes are transcribed, eventually inducing the production of replicable virus particles within 24 to 72 hours. pS1XX was prepared by introducing a non-reovirus epitope by redesigning the sequence from a STOP codon mutation to C-terminus at the amino acid position 251 of pS1Att, namely the S1 gene of RP116. The sequence after the STOP codon and the 763rd nucleotide can be redesigned to "fuse" an antigen or epitope fragment to the RP116 σ1 protein sequence. Specific restriction enzyme recognition sites were also designed between the amino acid position 251 and the C-terminus, allowing easy exchange of this portion of the σ1 peptide.

The produced replicable particles have been made more easily attachable and enterable to production cell lines BHK21, L929, Vero cells by decomposing the external capsid through chymotrypsin treatment at 50 µg/mL before infection.

Such a quick exchange reovirus reverse genetics system and the process of reovirus proliferation are illustrated in FIG. 2.

Furthermore, the amino acid sequence of the epitope used in the present invention, the construct amino acid sequence, and the codon-optimized construct DNA sequence are presented in Table 2.

**[Table 2]**

| **Epitope Name** | **Amino Acid Sequence** | **Construct Amino Acid Sequence** | **Codon Optimized Construct DNA Sequence (5'-3')** |
|---|---|---|---|
| **RBD** | NITNLCPFGE VFNATRFAS VYAWNRKRI SNCVADYSV LYNSASFSTF KCYGVSPTK LNDLCFTNV YADSFVIRG DEVRQIAPG QTGKIADYN YKLPDDFTG CVIAWNSNN LDSKVGGNY NYLYRLFRK SNLKPFERDI STEIYQAGST PCNGVEGFN CYFPLQSYG FQPTNGVGY QPYRVVVLS FELLHAPATV (SEQ NO: 3) | NITNLCPFGEVF NATRFASVYAWN RKRISNCVADYS VLYNSASFSTFK CYGVSPTKLNDL CFTNVYADSFVI RGDEVRQIAPGQ TGKIADYNYKLP DDFTGCVIAWNS NNLDSKVGGNY NYLYRLFRKSNL KPFERDISTEIYQ AGSTPCNGVEGF NCYFPLQSYGFQ PTNGVGYQPYR VVVLSFELLHAP ATV (SEQ NO: 3) | aatatcactaacttgtgtccgttcggcga ggtttttaatgcgaccaggtttgcttccgt gtacgcctggaacaggaaacggatctc caattgtgtcgccgattactccgtcttgtat aattcagcatctttcagcacgtttaaatgtt acggagtttcccccacaaaattgaatga cctttgctttacgaacgtctacgcggattc atttgtaatccggggggacgaagttagg caaattgcgccagggcagactggcaag atagctgactataattataaattgccggat gactttacgggctgtgtgattgcttggaa ctcaaataatctggactcaaaggtaggg ggaaattataactacctttacaggctgttc cggaagagtaatctgaagccattcgaaa gagatataagtacagagatctaccaagc tggaagcaccccctgcaatggtgttgaa ggattcaattgttatttcccattgcaatcct atggttttcaaccgacgaatggggtggg ataccaaccatatcgagttgtggttctca gtttcgagttgcttcatgctcctgcgacag tatgtggaccaaaaaaatctactaatctg gtgaagaataaatgcgtcaatttttaa (SEQ NO: 21) |
| **OVA**²⁵⁷⁻²⁶⁴ | SIINFEKL (SEQ NO: 4) | GGGGSGGGGSE QKLISEEDLSIINF EKLGGGGSSIINF EKLGGGGSSIINF EKLDYKDDDDK (SEQ NO: 14) | ggaggtggaggctcaggtggcggagg ttctgagcagaagttgatttcagaggaag atctgAGTATTATAAACTTC GAGAAGCTGGGTGGGGG AGGAAGTTCAATCATTAA TTTTGAAAAACTTGGAG GAGGTGGATCTTCCATAA TTAATTTTGAAAAGCTGg attataaggatgacgacgataagtga (SEQ NO: 22) |
| **OVA**³²³⁻³³⁹ | ISQAVHAAH AEINEAGR (SEQ NO: 5) | GGGGSGGGGSE QKLISEEDLISQA VHAAHAEINEA GRDYKDDDDK (SEQ NO: 15) | ggaggtggaggctcaggtggcggagg ttctgagcagaagttgatttcagaggaag atctgatttcacaggctgtgcatgctgca catgctgaaattaatgaggctggacgtg attataaggatgacgacgataagtga (SEQ NO: 23) |
| **Adpgk** | ASMTNMEL M (SEQ NO: 6) | GGGGSGGGGSE QKLISEEDLASM TNMELMGGGGS ASMTNMELMGG GGSASMTNMEL MDYKDDDDK (SEQ NO: 16) | ggaggtggaggctcaggtggcggagg ttctgagcagaagttgatttcagaggaag atctggctagcatgacgaacatggagct aatgggagggggcggaagtgcttctat gactaatatggaactgatgggaggtgga ggttctgcttcaatgaccaacatggaact tatggattataaggatgacgacgataagt ga (SEQ NO: 24) |
| **Rpl18** | KILTFDRL (SEQ NO: 7) | GGGGSGGGGSE QKLISEEDLKILT FDRLGGGGSKIL TFDRLGGGGSKI LTFDRLDYKDDD DK (SEQ NO: 17) | ggaggtggaggctcaggtggcggagg ttctgagcagaagttgatttcagaggaag atctgaagattctgacgtttgatcgtctgg ggggtggcggatctaagatattgacgtt cgatcgactgggaggaggtggctctaa gatcctaacattcgaccgtctagattataa ggatgacgacgataagtga (SEQ NO: 25) |
| **P15E** | KSPWFTTL (SEQ NO: 8) | GGGGSGGGGSE QKLISEEDLKSP WFTTLGGGGSK SPWFTTLGGGGS KSPWFTTLDYK DDDDK (SEQ NO: 18) | ggaggtggaggctcaggtggcggagg ttctgagcagaagttgatttcagaggaag atctgAAGTCACCTTGGTTT ACTACTCTCGGTGGGGG AGGAAGTAAATCGCCAT GGTTTACTACATTGGGAG GAGGTGGGTCTAAGTCA CCATGGTTCACGACATTG gattataaggatgacgacgataagtga (SEQ NO: 26) |
| **S21P2(1)** | PSKPSKRSFI EDLLFNKV (SEQ NO: 9) | GGGGSGGGGSE QKLISEEDLPSKP SKRSFIEDLLFNK VDYKDDDDK (SEQ NO: 19) | ggaggtggaggctcaggtggcggagg ttctgagcagaagttgatttcagaggaag atctgCCTAGTAAGCCTTCT AAGCGCAGCTTCATAGA AGATTTGTTGTTCAATAA GGTAgattataaggatgacgacgata agtga (SEQ NO: 27) |
| **S21P2(2)** | PSKPSKRSFI EDLLFNKV (SEQ NO: 10) | GGGGSGGGGSE QKLISEEDLPSKP SKRSFIEDLLFNK VGGGGSPSKPSK RSFIEDLLFNKV DYKDDDDK (SEQ NO: 20) | ggaggtggaggctcaggtggcggagg ttctgagcagaagttgatttcagaggaag atctgCCTAGTAAGCCTTCT AAGCGCAGCTTCATAGA AGATTTGTTGTTCAATAA GGTAGGTGGGGGAGGAA GTCCATCGAAGCCAAGT AAGCGTAGTTTCATTGAG GACCTCCTCTTCAACAA GGTTgattataaggatgacgacgata agtga (SEQ NO: 28) |

### Example 3. Preparation of ReoV-RBD

As a proof of concept for the reovirus vaccine platform, a recombinant reovirus ReoV+RBD (ReoV-RBD) was prepared by introducing the SARS-CoV-2 (Severe Acute Respiratory Syndrome Coronavirus 2) receptor binding domain (RBD) base sequence into the attenuated reovirus S1 gene after the 251st codon position, which expresses the σ1 protein of the reovirus and the codon-optimized SARS-CoV-2 RBD in a fusion protein form. The recombinant reovirus ReoV+RBD (ReoV-RBD), WT σ1 (WT ReoV) and reovirus with truncated σ1 (ReoV+Q251*) were produced in BHK21 cells and proliferated in L929 cells. In this process, the recombinant reovirus expressing the SARS-CoV-2 RBD from σ1 can be detected by SARS-CoV-2 neutralizing antibodies.

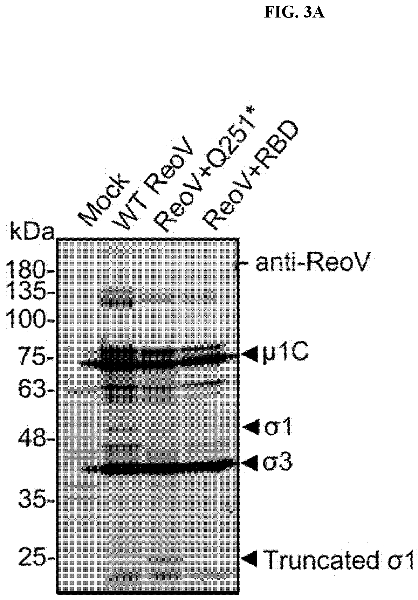

As shown in FIG. 3A, the wild-type reovirus (WT ReoV), reovirus with truncated σ1 (ReoV+Q251*), and reovirus introduced with RBD antigen (ReoV+RBD) were each infected into L929 cells and 96 hours later, each reovirus protein was detected in the cell lysate by anti-reovirus antibodies, as confirmed through Western blot results.

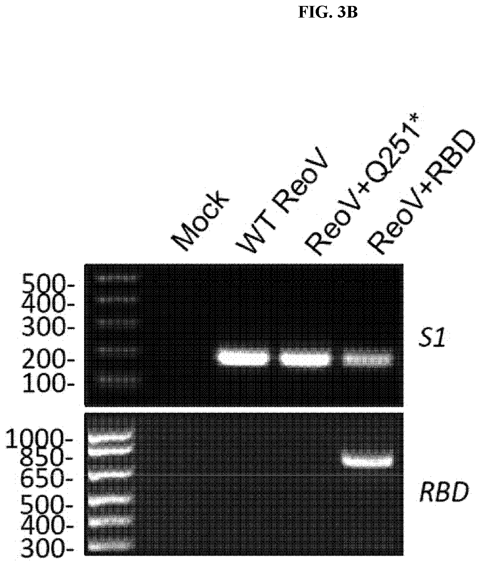

When each of the recombinant reoviruses was infected into L929 cells and genetic material extracted from them was subjected to RT-qPCR analysis using RBD gene-specific primers after four passages of the recombinant reovirus, as shown in FIG. 3B, the RBD gene was detected in the case of infection with ReoV+RBD. The primer sequences used for the RT-qPCR analysis are presented in Table 3 below.

**[Table 3]**

| Gene | Primer | 5' - 3' | SEQ NO: |
|---|---|---|---|
| S1 | S1 fwd | CACCCAGGGACTCGATGATG | 29 |
| FIGS. 3B, 4C, 4E | S1 rev | GCACCCAACTGGGTAACACT | 30 |
| RBD | RBD fwd | CAACTGAGCAAAGTTACGTGAGGGTAC AACCTACGGAATC | 31 |
| FIG. 3B | RBD rev | ATGAAATGCCCCAGTGCCGCGGGGTGG TCTGACCTCAAAAATTGACGCATTTATT CTTCAC | 32 |
| Epitope | S1-250-AvrII-F | GATTCTATCAACTCAAGGATAGGCGCAA TTGAGCCTAGG | 33 |
| FIGS. 4C, 4E | pBacT7-SacII-R | CAGTGCCGCGGGGTGGTCTGATCCTCA CGTGAAACTACGCGGGTA | 34 |

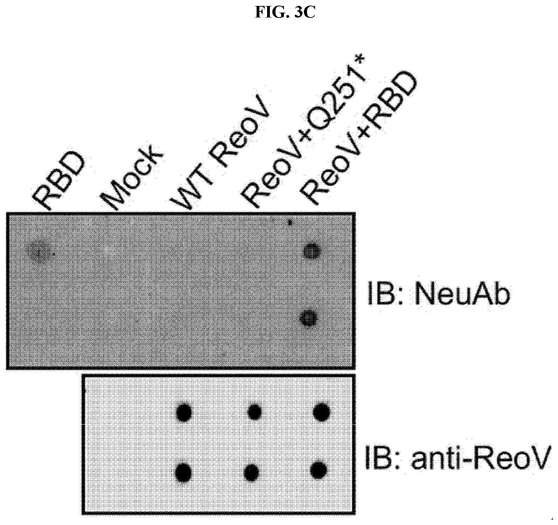

Furthermore, as a result of performing the dot blot method on the cell lysate obtained by infecting the L929 cells, as shown in FIG. 3C, it was confirmed that the recombinant RBD is expressed by the SARS-CoV-2 neutralizing antibody (NeuAb) (top) and anti-reovirus antibodies (bottom). The positive control recombinant RBD was prepared by Dr. John Bell's laboratory (Azad et al., Membranes (Basel). 2020 Aug 30;10(9):215) and the SARS-CoV-2 NeuAb (40592-MM57) was purchased from Sino Biological (Wayne, PA, USA) for use. The results indicate that the ReoV expressing the RBD provides an ideal neutralizing antibody for vaccine candidates.

### Example 4. Expression of Foreign Antigens Using Reovirus Attachment Protein Sigma-1 (σ1)

Specific mutation changes can be introduced by site-specific mutagenesis or replication to investigate the role or outcome of defined mutations within one or several portions of the reovirus genes, allowing the reovirus to be modified or undergo mutation. For instance, a specific amino acid mutation in the S4 gene of the reovirus encoding the sigma-3 protein is introduced into the virus vector, producing a modified reovirus having a sigma-3 protein of virus capsid with a specific mutation.

The attenuated reovirus RP116 of the present invention has its primary mutation in the S1 gene encoding the σ1 protein, STOP mutation from ⁷⁶³CAA (amino acid ²⁵¹Q-glutamine) to ⁷⁶³TAA (STOP codon), and this system produces a replicable RP116 reovirus with a truncated σ1 protein.

Depending on the characteristics of the gene fragment, substitution or insertion of a chosen specific epitope sequence at nucleotide positions 763 to 1416 is possible. Essentially, the substitution or insertion of this non-reovirus sequence replacing the spherical head structure of σ1 exposes a new epitope to the virus's capsid, generating an immune response against the non-reovirus antigen.

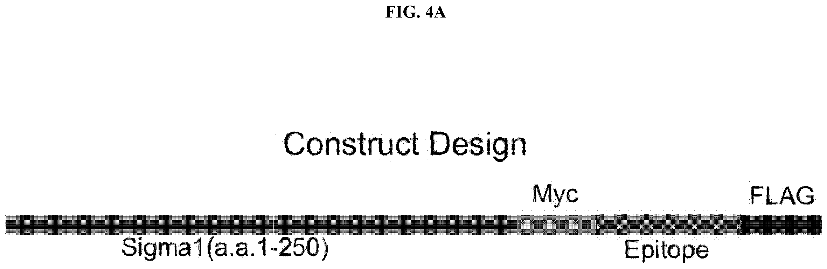

FIG. 4A represents a construct design of the recombinant attachment protein composition, where residues between the 251st amino acid and 455th amino acid of the σ1 protein can be replaced with various epitopes, such as Myc and FLAG tags for labeling purposes.

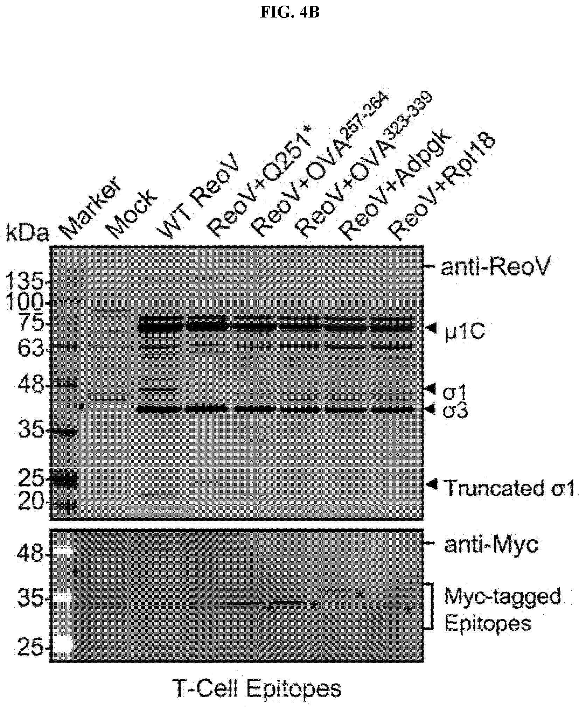

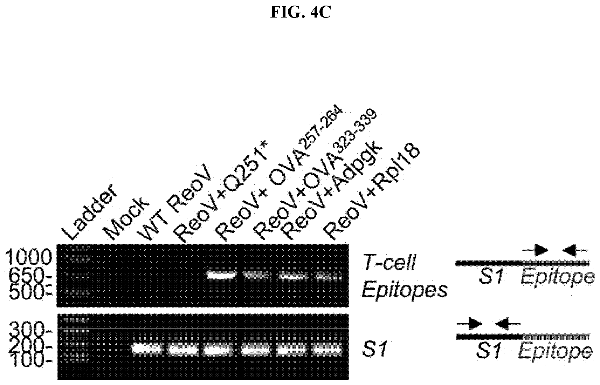

Reovirus was manipulated to express various CD4+ and CD8+ T cell epitopes fused with the σ1 protein using the "quick exchange" technique. As shown in FIG. 4B, the expression of various antigens introduced into the recombinant reovirus was confirmed by Western blot analysis, and as shown in FIG. 4C, the expression of the antigen introduced into the recombinant reovirus used in FIG. 4B was separated by DNA agarose gel and verified by performing RT-qPCR analysis using gene-specific primers.

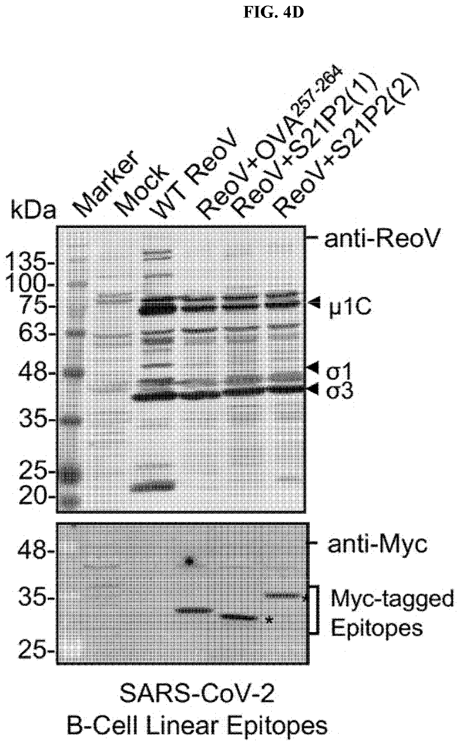

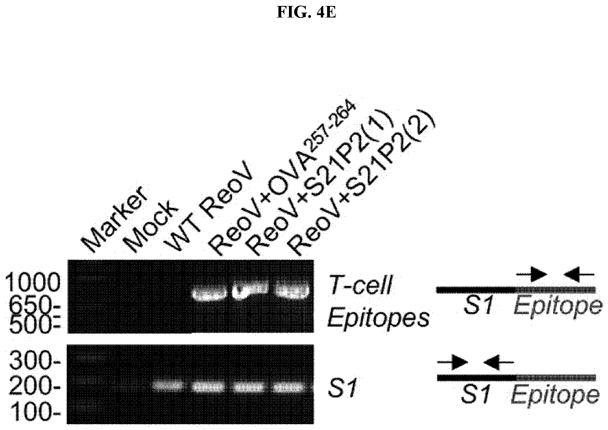

Also, reovirus was manipulated to express various B-cell epitopes fused with the σ1 protein using the quick exchange technique. As shown in FIG. 4D, the expression of various antigens introduced into the recombinant reovirus was confirmed by Western blot analysis, and as shown in FIG. 4E, the expression of the antigen introduced into the recombinant reovirus used in FIG. 4D was separated by DNA agarose gel and verified by performing RT-qPCR analysis using gene-specific primers.

Such examples demonstrate the diverse utility of the new reovirus vaccine platform expressing various foreign antigen epitopes, suggesting its potential use from the screening phase in the development of new vaccines by introducing various disease epitopes.

### Example 5. Confirmation of Antigen Expression Introduced in Cells Infected with ReoV-OVA

As represented in FIG. 4A, the Myc and FLAG tags positioned at the epitope of OVA²⁵⁷⁻²⁶⁴ in the prepared ReoV combined with ovalbumin (OVA)²⁵⁷⁻²⁶⁴ can be detected by immunocytochemical analysis.

To detect these, Vero cells grown on coverslips were infected with ReoV, ReoV+Q251*, and ReoV+OVA²⁵⁷⁻²⁶⁴ for 48 hours. Then, the cells were fixed in 3.7% PFA and reacted for 1 hour with anti-ReoV (1: 1000, rabbit serum), anti-Myc (1:200), and anti-FLAG (1:200), followed by a 45-minute incubation with secondary antibodies against anti-ReoV, rabbit Alexa Fluor 488, and secondary antibodies against anti-Myc or anti-FLAG, Alexa Fluor 594. The stained coverslips were mounted with Fluoromount G containing DAPI for nuclear counterstaining, and images were captured with an Olympus confocal microscope equipped with a 60X oil immersion objective (scale bar = 50 µm).

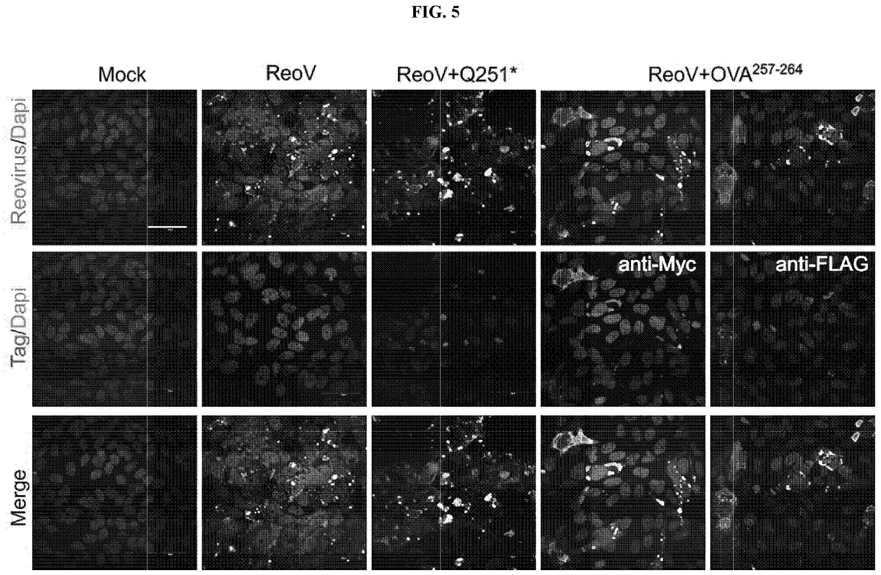

As a result, as depicted in FIG. 5, fluorescence (red) from the Myc and FLAG tags in cases infected with ReoV+OVA²⁵⁷⁻²⁶⁴ overlapped with the fluorescence (green) detected for the reovirus protein.

This indicates that the recombinant reovirus specifically expresses the antigen in infected cells, suggesting that the recombinant reovirus is infectious and can express the manipulated neoepitope in mammalian cells.

### Example 6. Expression and Detection of SARS-CoV-2 Linear B Cell Epitope

BHK21 cells were infected with either the wild-type (WT) or the manipulated ReoV (ReoV+p15E and ReoV-S21P2(2) expressing SARS-CoV-2 linear B cell epitope). Previous studies have verified that the SARS-CoV-2 linear B cell epitope produces neutralizing antibodies (Poh et al., Nat Commun. 2020 Jun 1;11(1):2806).

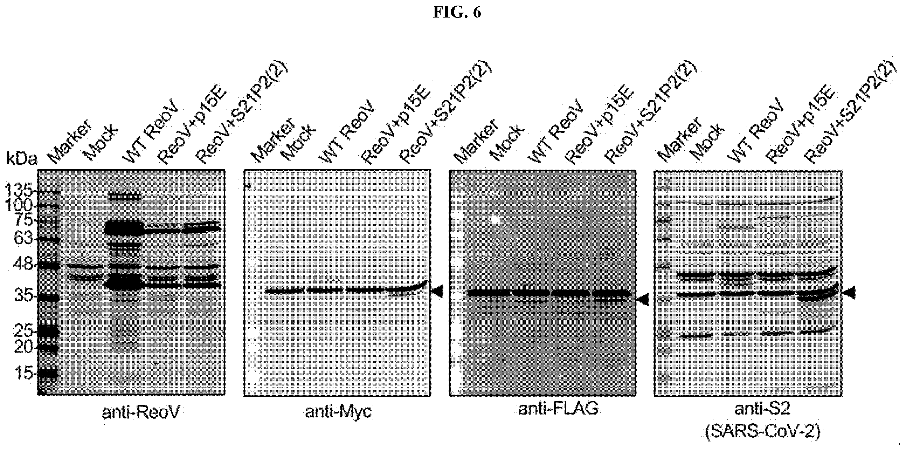

To confirm the expression of the SARS-CoV-2 linear B cell epitope, cells were lysed 48 hours post-infection and used for Western blot. As a result, as shown in FIG. 6, the reovirus protein could be detected in all infected samples. However, the Myc and FLAG tags were detected only in the ReoV manipulated with either p15E or the SARS-CoV-2 B cell epitope. Furthermore, after labeling with anti-S2 antibodies produced from the full S2 portion of the SARS-CoV-2 spike protein containing B cell epitope region of SARS-CoV-2 (provided by Sino Biological), a protein band with clear immunoreactivity was only identified in samples infected with ReoV+S21P2(2) (indicated by arrows).

These results suggest that the recombinant reovirus carrying the SARS-CoV-2 linear B cell epitope can induce a specific immune response against the epitope.

### Example 7. Verification of Stability of Recombinant Reovirus

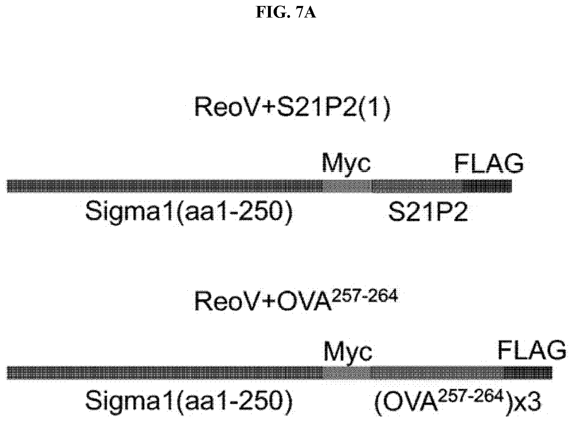

FIG. 7A represents a construct design of the recombinant σ1 protein configuration of the recombinant reoviruses ReoV+S21P2(1) and ReoV+OVA²⁵⁷⁻²⁶⁴.

Each epitope was designed to include the Myc and FLAG tags at the N-terminus and C-terminus, respectively, to verify the stable expression of the introduced epitope.

BHK21 cells were infected with the recombinant reovirus for 48 hours and analyzed by Western blot analysis, and were detected with both reovirus-specific antibodies (top) and specific antibodies against the FLAG tag (bottom) in recombinant reovirus. Antibodies recognizing GAPDH (anti-GAPDH) were used as a loading control.

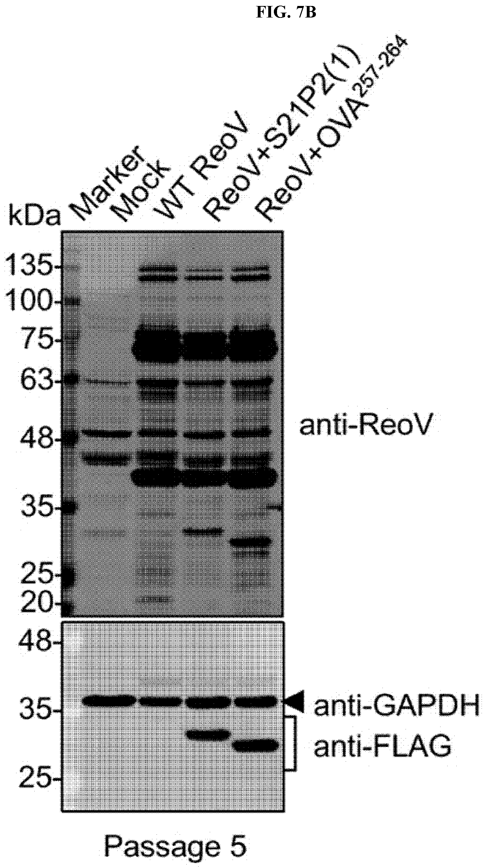

As a result, as shown in FIG. 7B, it was confirmed that a high concentration of antigen is detected by the FLAG tag, and it was found that the high concentration antigen can be stably expressed without mutations in the introduced epitope even after five passages.

The description of the present invention mentioned above is for illustrative purposes, and those skilled in the art to which the present invention belongs will understand that the present invention can easily be modified into other specific forms without changing its technical idea or essential characteristics. Therefore, the examples described above should be understood as being illustrative in all respects and not limited.

### [Industrial Applicability]

When an epitope of an antigenic protein is introduced into the truncated site of the attenuated reovirus sigma-1 protein according to the present invention, it is expected to be useful as a vaccine composition for cancer or an infectious disease. Thus, present invention has industrial applicability.

## Claims

1. A polypeptide comprising an amino acid sequence of an attenuated reovirus sigma-1 protein; and an amino acid sequence of an epitope of an antigen causing cancer or an infectious disease,
wherein the polypeptide comprises the 1 to 250th amino acid sequence of the attenuated reovirus sigma-1 protein truncated at the 251st amino acid position from the N-terminus; and
an amino acid sequence of an epitope of an antigen causing cancer or an infectious disease inserted at the amino acid position including and subsequent to the 251st from the N-terminus of the amino acid sequence of the attenuated reovirus sigma-1 protein.

2. The polypeptide of claim 1, wherein the attenuated reovirus sigma-1 protein is represented by the amino acid sequence of SEQ ID NO: 1.

3. The polypeptide of claim 2, wherein the attenuated reovirus sigma-1 protein is encoded by a base sequence represented by SEQ ID NO: 2.

4. The polypeptide of claim 1, wherein the epitope forms or constitutes a fusion protein at the carboxy-terminus of the attenuated reovirus sigma-1 protein.

5. The polypeptide of claim 1, further comprising one or more amino acid sequence selected from the group consisting of a linker (SEQ ID NO: 11), Myc protein (SEQ ID NO: 12), FLAG protein (SEQ ID NO: 13), and 2A peptide, both before and after the amino acid sequence of the epitope.

6. The polypeptide of claim 1, wherein the cancer is one or more selected from the group consisting of liver cancer, glioma, sarcoma, colon cancer, breast cancer, prostate cancer, melanoma, lung cancer, head and neck cancer, ovarian cancer, bladder cancer, stomach cancer, esophageal cancer, bile duct cancer, pancreatic cancer, cervical cancer, skin cancer, lymphoma, thyroid cancer, bone marrow cancer, endometrial cancer, kidney cancer, rectal cancer, and brain tumor.

7. The polypeptide of claim 1, wherein the infectious disease is one or more selected from the group consisting of coronavirus disease-19 (COVID-19), hepatitis C, influenza, human immunodeficiency virus (HIV)-induced acquired immune deficiency syndrome (AIDS), tuberculosis, severe acute respiratory syndrome (SARS), Middle East respiratory syndrome (MERS), infantile enteritis caused by rotavirus, and non-bacterial acute gastroenteritis caused by norovirus.

8. The polypeptide of claim 1, wherein the amino acid sequence of the epitope is one or more selected from the group consisting of SEQ ID NO: 3 to 10.

9. The polypeptide of claim 1, wherein the base sequence encoding the epitope is substituted or inserted at positions 763 to 1416 of from the 5' end of the base sequence encoding the attenuated reovirus sigma-1 protein.

10. The polypeptide of claim 1, wherein the epitope induces an immune response by producing neutralizing antibodies or inducing cell-mediated immunity in a host.

11. The polypeptide of claim 1, wherein the epitope is selected from the group consisting of CD4+ T cell, CD8+ T cell, and B-cell epitopes.

12. A virus vector based on attenuated reovirus, comprising a polynucleotide encoding the polypeptide of any one of claims 1 to 11.

13. A vaccine composition based on attenuated reovirus, comprising as an active ingredient, the polypeptide of any one of claims 1 to 11, a polynucleotide encoding the same, or the virus vector of claim 12.

14. The vaccine composition of claim 13, wherein the vaccine composition is for preventing or treating cancer or an infectious disease.

15. A method of preparing a vaccine composition based on the attenuated reovirus, comprising the following steps:
a) preparing a reverse genetics virus vector comprising a base sequence encoding an attenuated reovirus sigma-1 protein represented by SEQ ID NO: 1; and
b) introducing a polynucleotide into the vector, wherein the polynucleotide encodes an attenuated reovirus sigma-1 protein and an epitope of an antigen causing cancer or an infectious disease, which is expressed as a fusion, by substituting or inserting a base at positions 763 to 1416 of the base sequence encoding the sigma-1 protein, so as to comprise a base sequence encoding the epitope of the antigen causing cancer or an infectious disease.

16. The method of claim 15, further comprising the step of
c) producing and proliferating the vector based on the attenuated reovirus comprising the polynucleotide from the step b) in one or more cell selected from the group consisting of BHK21, L929, HEK293, CHO, PER.C6, HeLa, and Vero cells.

17. A method for preventing or treating cancer or an infectious disease, comprising the step of administering the vaccine composition of claim 13 to a subject in need thereof.

18. A use of the vaccine composition of claim 13 for preventing or treating cancer or an infectious disease.

19. A use of the polypeptide of claim 1, a polynucleotide encoding the same, or a virus vector comprising the polynucleotide for preparing a vaccine for preventing or treating cancer or an infectious disease.
